(19) Europäisches Patentamt
European Patent Office
Office européen des brevets

(11) Veröffentlichungsnummer: **0 135 018**
**B1**

(12) **EUROPÄISCHE PATENTSCHRIFT**

(45) Veröffentlichungstag der Patentschrift: **19.10.88**

(21) Anmeldenummer: **84108316.5**

(22) Anmeldetag: **14.07.84**

(51) Int. Cl.4: **C 11 C 3/00**, A 61 K 7/00, A 61 K 47/00

(54) **Kosmetisch-pharmazeutische Ölkomponenten.**

(30) Priorität: **22.07.83 DE 3326455**

(43) Veröffentlichungstag der Anmeldung: **27.03.85 Patentblatt 85/13**

(45) Bekanntmachung des Hinweises auf die Patenterteilung: **19.10.88 Patentblatt 88/42**

(84) Benannte Vertragsstaaten: **AT BE CH DE FR GB IT LI NL SE**

(56) Entgegenhaltungen:
**DE-A- 1 806 694**
**US-A- 3 035 069**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Scheuffgen, Ingeborg**
**Spulgasse 3**
**D-4040 Neuss (DE)**
Erfinder: **Meffert, Alfred, Dr.**
**Marie-Curie-Strasse 10**
**D-4019 Monheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 135 018 B1

Jouve, 18, rue St-Denis, 75001 Paris, France

**Beschreibung**

Gegenstand der Erfindung sind neue Ölkomponenten für pharmazeutische Anwendungen.

Ölige Flüssigkeiten natürlicher oder synthetischer Herkunft bilden die Grundlage zahlreicher kosmetischer und pharmazeutischer Zubereitungen wie Cremes, Salben, Öle und Stiftpräparate. Sie erfüllen in derartigen Mitteln verschiedene Aufgaben : Sie sollen der Haut nach der Anwendung Glätte und Geschmeidigkeit verleihen, ihr nach dam Waschen Fettstoffe zuführen, ihr Austrocknen verhindern und als Lösungsmittel und Träger der verschiedenen kosmetischen und dermatologischen Wirkstoffe und/oder Pigmente dienen.

Als Ölkomponenten werden sowohl natürliche, pflanzliche und tierische Öle wie z. B. Olivenöl, Erdnußöl, Mandelöl, Jojobaöl, Rizinusöl, Nerzöl, Spermöl als auch mineralische Öle, z. B. Paraffinöle, eingesetzt. Brauchbar sind auch synthetische Ester natürlicher Fettsäuren und einwertiger oder mehrwertiger Alkohole sowie modifizierte Naturstoffe wie hydriertes Squalen oder hydrierte Terpene.

In US-A 3 035 069 werden Alkoholysate epoxidierter Triglyceridöle als Schmieröl- und Schmierfettzusätze und als Vorprodukte für die Herstellung von Polyurethanharzen beschrieben.

Die natürlichen, pflanzlichen oder tierischen Öle enthalten nennenswerte Mengen ungesättigter Verbindungen. Dies führt dazu, daß sie unter dem Einfluß von Wärme, Luftsauerstoff, Licht und Feuchtigkeit die Eigenschaften verändern.

Gesättigte mineralische Öle und synthetisch hergestellte Ölkomponenten sind zwar gegen Autoxidation und Ranzidität stabil, sind aber oft hautfremd und werden in kosmetischen Präparaten daher auch als unangenehm empfunden. Sie führen häufig zu einer unerwünschten Glanzbildung auf der Haut und behindern deren Wasserdampfdurchlässigkeit. Sie haben aufgrund ihrer geringeren Polarität auch ein geringeres Lösevermögen für kosmetische und dermatologische Wirkstoffe.

Wenn man natürliche Öle durch Hydrierung absättigt, verlieren sie ihren klarflüssigen Charakter und es entstehen steife Fette, die als Ölkomponenten unbrauchbar sind. Triglyceride auf Basis kürzerer, gesättigter Fettsäuren, die noch flüssig sind, weisen eine für viele Anwendungen zu niedrige Viskosität auf. Zur Herstellung dekorativer Kosmetikpräparate, speziell wasserfreier Zubereitungen in Stift- und Cremeform, werden aber Öle benötigt, die sich durch eine gewisse Zähigkeit bei gleichzeitig niedrigem Stockpunkt auszeichnen. Weiterhin sollen kosmetische Ölkomponenten reizlos haut- und schleimhautverträglich sein.

Es bestand daher die Aufgabe, Ölkomponenten aufzufinden, welche die genannten nachteile nicht aufweisen und die sowohl eine gute Haut- und Schleimhautverträglichkeit, eine hohe Stabilität gegen Ranzidität, ein gutes Lösevermögen als auch eine hohe Viskosität bei niedrigem Stockpunkt aufweisen.

Es wurde gefunden, daß diese Forderungen weitgehend erfüllt werden durch Ölkomponenten, die durch Umsetzung epoxidierter triglyceridöle mit einwertigen Alkoholen mit 1-4 C-Atomen erhältlich sind.

Epoxidierte Triglyceride sind als sogenannte Epoxidweichmacher bekannte und im Handel erhältliche Produkte. Sie werden durch Epoxidation ungesättigter Öle wie z. B. von Sojaöl, Leinöl, Tallöl, Baumwollsaatöl, Erdnußöl, Palmöl, Sonnenblumenöl, Rüböl oder Klauenöl, z. B. nach dem in J. Am. Chem. Soc. 67, März 1945, Seiten 412-414 beschriebenen Verfahren mit Peressigsäure erhalten. Durch die Epoxidation werden, je nach dem Umsetzungsverhältnis, die Doppelbindungen ganz oder teilweise in Oxiranringe überführt. Geeignet sind Triglyceride mit einer Iodzahl von 50-150, die bei weitgehender Epoxidation in Epoxidate mit einem Gehalt von 3-10 Gew.-% Epoxidsauerstoff überführt werden. Besonders geeignet für die weitere Umsetzung mit einwertigen Alkoholen sind Epoxidate mit einem Gehalt von 4-8 Gew.-% Epoxidsauerstoff. Die Umsetzung mit einwertigen Alkoholen mit 1-4 C-Atomen in Gegenwart eines sauren Katalysators, bevorzugt von konzentrierter Schwefelsäure, führt zur Öffnung der Oxiranringe unter Ausbildung von $HO{-}\overset{|}{\underset{|}{C}}{-}\overset{|}{\underset{|}{C}}{-}OR$-Gruppen. Die Umsetzung wird mit hohem Alkoholüberschuß durchgeführt, um die quantitative Öffnung aller Oxirangruppen sicherzustellen. Bevorzugt wird ein Überschuß von 4-10 Mol Alkohol pro Mol Epoxidsauerstoff eingesetzt. Als einwertige Alkohole können z. B. Methanol, Ethanol, Propanol-1, Isopropanol und Butanol oder isobutanol eingesetzt werden.

Auf diese Weise werden flüssige, relativ hochviskose, gegen Autoxidation und Ranzidität sehr stabile, haut- und schleimhautverträgliche Ölkomponenten erhalten. Die Produkte haben ein gutes Lösevermögen für kosmetische und dermatologische Wirkstoffe und ein gutes Dispergiervermögen für Pigmente. Sie eignen sich daher vorzüglich zur Herstellung kosmetischer und pharmazeutischer Zubereitungen. Besonders vorteilhaft ist die Verwendung solcher Ölkomponenten für die Herstellung dekorativer kosmetischer Stiftpräparate wie z. B. Lippenstifte, Make-up-Stifte, Lidstrich- und Lidschattenstifte. Solche Stifte werden aus Mischungen von kosmetischen Ölen und Wachsen aufgebaut. Zur Einstellung der geforderten Konsistenz und Stabilität werden außerdem Fettalkohole, Emulgatoren, z. B. Fettsäurepartialglyceride, Wollfett, Paraffinkohlenwasserstoffe sowie die kosmetischen Pigmente und ggf. kosmetische und dermatologische Wirkstoffe zugesetzt. Solche Stiftpräparate sollen zwar eine feste Konsistenz und Wärmestabilität aufweisen, gleichzeitig soll aber ein guter Abrieb gewährleistet sein. Dies erfordert die Zugabe flüssiger Ölkomponenten in relativ hohen Mengen. Diese Ölkomponenten sollen auch bei hohen Anteilen nicht aus der Stiftmasse ausölen. Das für diesen Zweck gebraüchliche Rizinusöl hat den

Nachteil einer geringen Oxidationsstabilität.

Überraschend hat sich gezeigt, daß die erfindungsgemäßen Ölkomponenten das Rizinusöl in kosmetischen Stiftpräparaten gut ersetzen können. Dies gilt besonders für das Umsetzungsprodukt von epoxidiertem Sojaöl, welches 6-7 Gew.-% Epoxidsauerstoff enthält, mit Methanol, insbesondere dann, wenn dieses Umsetzungsprodukt eine Iodzahl unter 20 aufweist. Es kann in erfindungsgemäßen Stifpräparaten in Menge von 5-60 Gew.-% eingearbeitet werden. Bevorzugt enthalten Stiftpräparate 20-50 Gew.-% der erfindungsgemäßen Ölkomponenten. Dabei werden Stiftpräparate von besonders guter, gegenüber Zubereitungen mit Rizinusöl verbesserte Oxidationsstabilität, vergleichbarer Konsistenz, Pigmentverteilung, Schleimhautverträglichkeit und leicht verbesserter Hautverträglichkeit erhalten.

Auch in anderen kosmetischen und dermatologischen Zubereitungen, z. B. in Salben, Cremes, Hautölen, Lotionen, Badeölen, in Haarpflegemitteln, Duftwässern und Deodorantien kommen die vorzüglichen kosmetischen Eigenschaften der erfindungsgemäßen Ölkomponenten voll zu Geltung.

Die nachfolgenden Beispiele sollen den Gegenstand der Erfindung weiter erläutern.

## Beispiele

1. Umsetzungsprodukt von Sojaölepoxid mit Methanol

In einem Reaktionskessel mit Rückflußkühlung wurden 657 kg Methanol und 1,7 kg $H_2SO_4$ (konz.) vorgelegt und zum Sieden unter Rückfluß (ca. 65 °C) erwärmt. In die siedende Lösung wurden 990 kg eines käuflichen Sojaölepoxids (Edenol [(R)]D 81) mit einem Gehalt von 6,3 Gew.-% Epoxid-Sauerstoff nach und nach eingegeben. Durch die Reaktionswärme blieb die Lösung auf Siedetemperatur. Nach beendeter Zugabe wurde das Reaktionsgemisch noch 3 Stunden bei 65 °C nachgerührt. Dann wurde mit 6 kg einer 30 Gew.-%igen Lösung von Natriummethylat in Methanol neutralisiert und das überschüssige Methanol ca. 1 000 kg des Umsetzungsproduktes als hellgelbe klare Flüssigkeit zurück.

Das Produkt wies die folgenden technischen Daten aus :

| | |
|---|---|
| Säurezahl (nach DGF-C-V-2) : | 0,2 |
| Verseifungszahl (nach DIN-53401) : | 162 |
| Iodzahl (nach DGF-C-V-11b) : | 14,5 |
| Hydroxylzahl (nach DGF-C-V-17a) : | 207 |
| Dichte 20 °C (nach DGF-C-IV-2) : | 1,009 |
| Viskosität (20 °C) : (Brookfield, Spindel, 2, 10 Upm) | 2,36 Pa.s |

2. Prüfung der Autoxidationsneigung im Vergleich zu Rizinusöl.

Das Umsetzungsprodukt gemäß Beispiel 1 wurde in reiner Form sowie in einer Stiftgrundmasse mit einem Anteil an 50 Gew.-% des Produkts nach Beispiel 1 über einen Zeitraum von 40 Tagen bei 20 °C im offenen Gefäß gelagert. In dieser Zeit stieg infolge von Autoxidationsvorgängen die Peroxidzahl an. Vergleichend wurde Rizinusöl in reiner Form sowie in der gleichen Stiftgrundmasse mit einem Anteil von 50 Gew.-% des Rizinusöls dem Lagertest unterworfen.

Die Stiftmassen wurden zusätzlich in einer mit Anti-oxidans versetzten Form dem Lagertest unterzogen. Als Antioxidans wurde das Systems aus 0,02 Gew.-% Butylhydroxyanisol (BHA) und 0,0016 Gew.-% Citronensäure eingesetzt.

Die Stiftgrundmasse hatte folgende Zusammensetzung :

| | |
|---|---|
| Bienenwachs | 2,5 Gew.-% |
| Carnaubawachs | 7,5 Gew.-% |
| Candellilawachs | 7,5 Gew.-% |
| Mikrowachs (MP : 72 °C) | 4,0 Gew.-% |
| Cetylalkohol | 2,5 Gew.-% |
| Glycerinmonoricinoleat | 13,0 Gew.-% |
| Ricinusöl/oder Prod. nach Beisp. 1 | 50,0 Gew.-% |
| Paraffinöl dickflüssig | 2,5 Gew.-% |
| 2-Octyl-dodecanol-1 | 10,5 Gew.-% |

Die Bestimmung der Peroxidzahl erfolgte nach Wheeler (DGF-Einheitsmethode C-VI 6a).

(Siehe Tabelle Seite 4 f.)

| Substrat | POZ (Milliäquivalent Sauerstoff pro kg) Anfangswert | nach 20 Tagen | nach 40 Tagen |
|---|---|---|---|
| Produkt gemäß Beispiel 1 (ohne Antioxidans) | 2,8 | 4,5 | 7,3 |
| Ricinusöl (ohne Antioxidans) | 2,8 | 9,9 | 23,6 |
| Stiftgrundmasse mit Prod. Beispiel 1 (ohne Antioxidans) | 3,8 | 7,8 | 12,4 |
| Stiftgrundmasse mit Ricinusöl (ohne Antioxidans) | 6,3 | 11,0 | 16,4 |
| Stiftgrundmasse mit Prod. Beispiel 1 + Antioxidans | 4,4 | 5,8 | 6,3 |
| Stiftmasse mit Ricinusöl, + Antioxidans | 5,6 | 7,8 | 12,5 |

Aus der Entwicklung der Peroxidzahl war zu erkennen, daß das Produkt nach Beispiel 1 sowie damit angefertigte Zubereitungen gegen Autoxidation (Ranzidität) stabiler sind.

3. Prüfung der Hautverträglichkeit und Vergleich mit Rizinusöl.

Das Produkt nach Beispiel 1 wurde unverdünnt mittels Okklusionsmethode an Kaninchen (Patch-Test, in Anlehnung an Fed. Register Vol., No. 187, & 1500.41 (27.09.1973)) geprüft. Die gleiche Prüfung erfolgte mit Rizinusöl.

Ergebnis

Prod. Beispiel 1 führte bei allen Versuchstieren nach 24-stündiger Kontaktzeit zu leichten bis deutlichen Rötungen der Haut, die in 4-5 Tagen völlig abklangen.

Rizinusöl führte bei allen Versuchstieren nach 24-stündiger Kontaktzeit zu deutlichen bis starken Rötungen der Haut, begleitet von leichter Schwellung bei einigen Tieren. Die langsam abklingenden Unverträglichkeitsreaktionen waren am 4. und 5. Tag des Versuchs bei einem Teil der Tiere noch wahrnehmbar, nach 7 Tagen wurden bei keinem der Kaninchen mehr Befunde festgestellt.

4. Anwendungsbeispiele

4.1 Lippenstift

| | |
|---|---|
| Oleylalkohol (HD-Butanol$^{(R)}$)$^{2)}$ | 20,0 Gew.-% |
| Produkt nach Beispiel 1 | 25,0 Gew.-% |
| Bienenwachs | 5,0 Gew.-% |
| Carnaubawachs | 8,0 Gew.-% |
| Ozokerit (FP : 70-72 °C) | 11,0 Gew.-% |
| Candelillawachs | 3,0 Gew.-% |
| Wollfett, wasserfrei | 8,0 Gew.-% |
| Paraffinöl, dickflüssig | 13,0 Gew.-% |
| $TiO_2$C 47051$^{1)}$ | 5,3 Gew.-% |
| Red 300404 | 1,7 Gew.-% |

Das Produkt ließ sich leicht verarbeiten, die Pigmente lagen gut verteilt vor. Aussehen und Stabilität und Steigschmelzpunkt (70 °C) sind identisch einem Stift, bei welchem Rizinusöl anstelle von Produkt nach Beispiel 1 eingesetzt wurde.

4.2 Lidstrichstift

| | |
|---|---|
| Cetylalkohol | 5,0 Gew.-% |
| Produkt nach Beispiel 1 | 20,0 Gew.-% |
| Oleylalkohol (HD-Butanol(R))[2] | 29,0 Gew.-% |
| Glycerinmonostearat (Cutina(R)GMS)[2] | 5,0 Gew.-% |
| Bienenwachs | 2,0 Gew.-% |
| Candelillawachs | 6,0 Gew.-% |
| Carnaubawachs | 6,0 Gew.-% |
| Ozokerit, (FP 70-72 °C) | 15,0 Gew.-% |
| Paraffinöl, dickflüssig | 2,0 Gew.-% |
| Pigmentfarbe Ariabel Braun 300402[1] | 10,0 Gew.-% |

Das Produkt ließ sich leicht verarbeiten, das Pigment lag gut verteilt vor. Aussehen, Stabilität und Steigschmelzpunkt (68,5 °C) sind vergleichbar einem analogen Stift, bei welchem Rizinusöl anstelle von Produkt nach Beispiel 1 eingesetzt wurde.

4.3 Lidschattenstift

| | |
|---|---|
| Laurinsäurehexylester (Cetiol(R)A)[2] | 35,0 Gew.-% |
| Produkt nach Beispiel 1 | 10,0 Gew.-% |
| Bienenwachs | 6,0 Gew.-% |
| Carnaubawachs | 5,0 Gew.-% |
| Candelillawachs | 10,0 Gew.-% |
| Fischsilber-Pigment (Merck) | 30,0 Gew.-% |
| Pigmentfarbe Dark Blue 300308 [1] | 4,0 Gew.-% |

Das Produkt ließ sich leicht verarbeiten, die Pigmente lagen gut verteilt vor: Aussehen, Stabilität und Steigschmelzpunkt (68,5 °C) sind vergleichbar einem Stift, bei welchem Rizinusöl anstelle von Produkt nach Beispiel 1 eingesetzt wurde.

[1] Die Pigmentfarben sind erhältlich bei Fa. Williams, Hounslow.
[2] Diese Produkte sind erhältlich von Fa. Henkel KGaA, Düsseldorf.

**Patentansprüche**

1. Ölkomponenten für pharmazeutische Anwendungen, dadurch gekennzeichnet, daß sie aus Umsetzungsprodukten epoxidierter Triglyceridöle mit einwertigen Alkoholen mit 1-4 C-Atomen bestehen.
2. Ölkomponenten nach Anspruch 1, dadurch gekennzeichnet, daß sie aus epoxidierten Triglyceriden mit einem Gehalt von 4-8 Gew.-% Epoxidsauerstoff hergestellt sind.
3. Ölkomponenten nach Anspruch 1-2, dadurch gekennzeichnet, daß die epoxidierten Triglyceridöle mit Methanol umgesetzt sind.
4. Ölkomponente nach Anspruch 1-3, dadurch gekennzeichnet, daß sie aus dem Umsetzungsprodukt eines epoxidierten Sojaöls, welches 6-7 Gew.-% Epoxidsauerstoff enthält, mit Methanol besteht.
5. Verwendung der Ölkomponenten nach Anspruch 1-4 zur Herstellung kosmetischer und pharmazeutischer Zubereitungen.
6. Kosmetische Stiftpräparate, gekennzeichnet durch einen Gehalt an Ölkomponenten nach Anspruch 1-4.

**Claims**

1. Oil components for pharmaceutical applications, characterized in that they consist of reaction products of epoxidized triglyceride oils with monohydric alcohols containing from 1 to 4 carbon atoms.
2. Oil components as claimed in claim 1, characterized in that they are produced from epoxidized triglycerides containing from 4 to 8 % by weight of epoxide oxygen.
3. Oil components as claimed in claims 1 and 2, characterized in that the epoxidized triglyceride oils are reacted with methanol.
4. An oil component as claimed in claims 1 to 3, characterized in that it consists of the reaction

product of an epoxidized soya bean oil containing from 6 to 7 % by weight of epoxide oxygen with methanol.

5. The use of the oil components claimed in claims 1 to 4 for the production of cosmetic and pharmaceutical preparations.

6. Cosmetic stick preparations, characterized by a content of the oil components claimed in claims 1 to 4.

**Revendications**

1. Composants huileux pour applications pharmaceutiques, caractérisés en ce qu'ils consistent en des produits de réaction d'huiles triglycéridiques époxydées avec des alcools monovalents ayant de 1 à 5 atomes de carbone.

2. Composants huileux selon la revendication 1, caractérisés en ce qu'on les prépare à partir de triglycérides époxydés ayant une teneur en oxygène en fonction époxy allant de 4 à 8 % en poids.

3. Composants huileux selon la revendication 2, caractérisés en ce qu'on fait réagir les huiles triglycéridiques époxydées avec du méthanol.

4. Composants huileux selon l'une quelconque des revendications 1 à 3, caractérisés en ce qu'ils consistent en le produit de réaction avec le méthanol d'une huile de soja époxydée qui contient de 6 à 7 % en poids d'oxygène en fonction époxy.

5. Utilisation des composants huileux selon l'une quelconque des revendications 1 à 4, pour la préparation de compositions cosmétiques et pharmaceutiques.

6. Compositions de bâtons cosmétiques caractérisés par une teneur en composants huileux selon l'une quelconque des revendications 1 à 4.